(19) Europäisches Patentamt
European Patent Office
Office européen des brevets

(11) Publication number: 0 186 426 B1

# (12) EUROPEAN PATENT SPECIFICATION

(45) Date of publication of patent specification: 12.09.90

(51) Int. Cl.[5]: C 07 D 257/04

(21) Application number: 85309218.7

(22) Date of filing: 18.12.85

## (54) Tetrazolyl compounds and their pharmaceutical use.

(30) Priority: 20.12.84 GB 8432144

(43) Date of publication of application:
02.07.86 Bulletin 86/27

(45) Publication of the grant of the patent:
12.09.90 Bulletin 90/37

(84) Designated Contracting States:
AT BE CH DE FR GB IT LI NL SE

(56) References cited:
EP-A-0 134 111

TETRAHEDRON, vol. 40, no. 12, June 1984, pages 2219-2224, Pergamon Press, Oxford, GB; R.W. HOFFMANN et al.: "Acyclic stereocontrol in the addition of gamma-alkylthio-allylboronates to aldehydes"

(73) Proprietor: Lilly Industries Limited
Lilly House Hanover Square
London W1R 0PA (GB)

(72) Inventor: Baker, Stephen Richard
16 Whitley Road
Yateley Camberley Surrey (GB)

(74) Representative: Hudson, Christopher Mark et al
Erl Wood Manor
Windlesham Surrey GU20 6PH (GB)

EP 0 186 426 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel compounds and their use as pharmaceuticals.

The compounds of the invention are of the formula

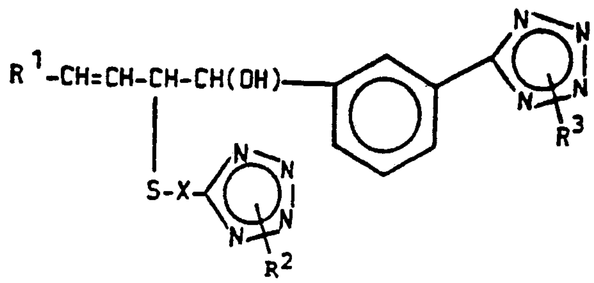

(I)

in which $R^1$ is $C_{7-20}$alkyl or $C_{7-20}$ alkenyl containing 1 to 3 double bonds, $R^2$ and $R^3$ are each hydrogen or a protecting group, and X is $C_{1-6}$ alkylene; and salts thereof.

The compounds of the invention, with the exception of compounds in which $R^2$ or $R^3$ is a protecting group which are intermediates in the preparation of the remaining compounds, have been shown to be pharmacologically active in tests which demonstrate their antagonist effect on leukotriene receptors and indicate their use in the treatment of allergy disorders.

In the above general formula, $R^1$ can be an alkyl group containing from 7 to 20 carbon atoms, and is preferably alkyl containing from 8 to 13 carbon atoms, such as for example 10 to 13 carbon atoms. The alkyl group is preferably straight chained. When $R^1$ is alkenyl it contains from 7 to 20 carbon atoms and preferably from 8 to 13 carbon atoms, such as for example 10 to 13 carbon atoms. Preferably the alkenyl group is unbranched and contains 1 or 3 double bonds. Particularly preferred alkenyl groups are those of formula $R^4CH=CH—$ where $R^4$ is $C_{6-11}$ alkyl or $CH_3(CH_2)_4CH=CHCH_2CH=CH_2—$.

It will be appreciated that such double bonds, and the double bond between the 3 and 4 carbon atoms, provide opportunities for cis-trans isomeric forms. It is preferred that the $R^1$ group is arranged in trans configuration about the double bond at the 3 and 4 carbon atoms. It will also be appreciated that the compounds of formula (I) possess chiral centres at the carbon atoms bearing the hydroxyl and thio groups and, accordingly, stereoisomeric forms exist R,R; S,S; R,S; and S,R. All such stereoisomers, and racemic mixtures thereof, are included within the scope of the invention. The preferred compounds are of S,R configuration. Isomers can be isolated from racemic mixtures by conventional methods such as by the preparation of diastereoisomers with subsequent liberation of the enantiomers or, alternatively, can be prepared by methods devised to give the pure isomer.

Examples of particularly preferred groups are those in which the moiety $R^1CH=CH$ in formula (I) above is

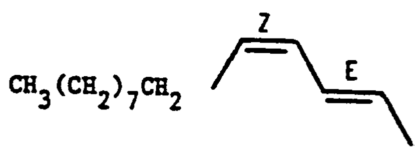

and

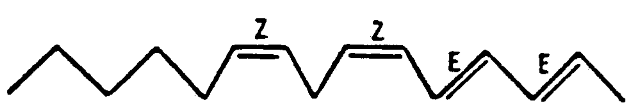

The "X" group in formula (I) above is an alkylene group containing 1 to 6 carbon atoms which can be branched or unbranched and is preferably of the formula $—(CH_2)_m—$ where m is 1, 2 or 3.

When $R^2$ or $R^3$ is a protecting group it can be any of the well known protecting groups employed for the purpose of protecting the tetrazolyl radical and such groups include optionally substituted trityl and benzhydryl, and optionally substituted benzyl for example p-methoxybenzyl, or a silyl group for example t-butyldiphenylsilyl. The preferred intermediates are those in which $R^2$ is hydrogen and $R^3$ is a protecting group. As is well known the tetrazolyl group exhibits tautomerism and the position of attachment of the protecting group will depend on its size, though generally the point of attachment is at the 2-position.

The compound of formula (I) bears either one or two acidic functions, depending on whether the $R^2$ or $R^3$ substituent is a protecting group or hydrogen. In the latter case the free tetrazolyl groups provide two

acidic functions. Base addition salts of these compounds can thus be prepared and these are included as part of the present invention. Examples of such salts are those derived from ammonium hydroxide and alkali and alkaline earth metal hydroxides, carbonates and bicarbonates, as well as salts derived from aliphatic and aromatic amines, aliphatic diamines and hydroxy alkylamines. Bases especially useful in the preparation of such salts include ammonium hydroxide, potassium carbonate, sodium bicarbonate, calcium hydroxide, methylamine, diethylamine, ethylene diamine, cyclohexylamine and ethanolamine. The potassium and sodium salt forms together with other pharmaceutically acceptable salts are particularly preferred, but it is to be understood that other non-pharmaceutical salts are involved in the invention since they may be useful for identification, characterisation or purification of the free compound.

A particular group of compounds according to formula (I) above are those of the formula

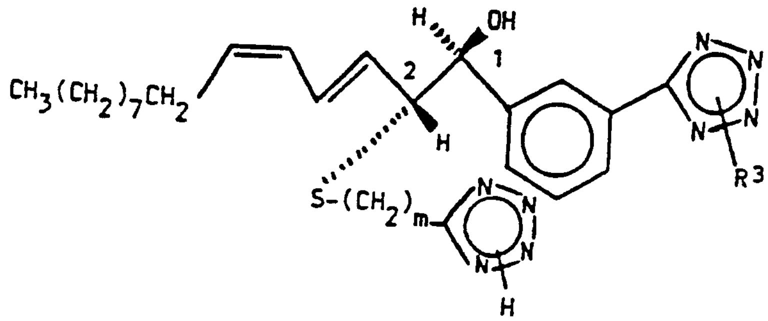

in which $R^3$ is hydrogen or a protecting group and m is 1, 2 or 3; and salts thereof. Such compounds exhibit 1S, 2R chirality.

The invention also includes a process for producing a compound of general formula (I) which comprises reacting a compound of formula

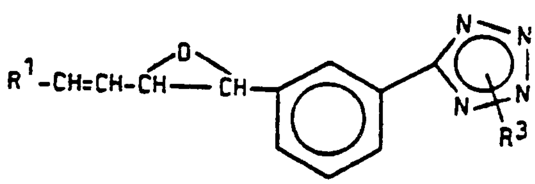

(II)

in which $R^1$ is as defined above and $R^3$ is a protecting group, with a thiol of formula

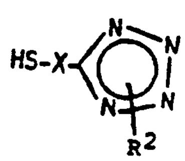

(III)

in which $R^2$ is hydrogen or a protecting group, optionally followed by removal of the $R^2$ or $R^3$ protecting group. The reaction is generally carried out in the presence of a base, preferably a strong base such as potassium t-butoxide in an organic solvent such as for example t-butanol at a temperature of from 0°C to 60°C.

Intermediate compounds of formula (II) may be prepared by the Wittig reaction of a phosphonium salt

of formula $R^4CH_2P^+Ph_3Br^-$, $R^4$ being an appropriate alkyl or alkenyl group, in the presence of a base such as butyl lithium, with an aldehyde of formula (IV) or (V)

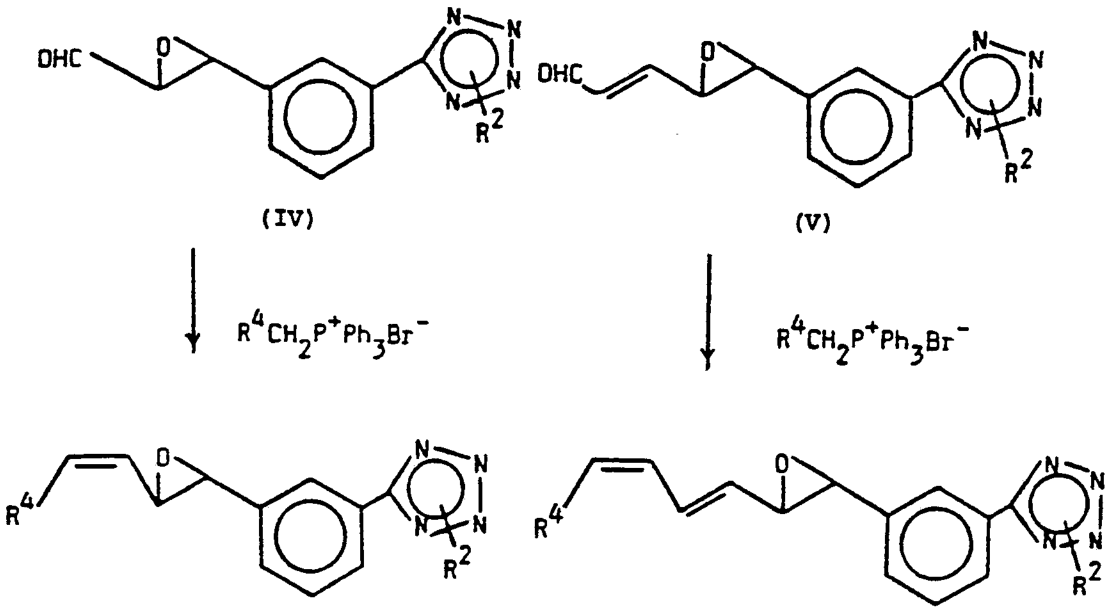


where $R^2$ is a protecting group. The reaction is generally carried out in an inert organic solvent such as for example, tetrahydrofuran, at a temperature of from −110°C to 0°C.

Compounds of formula (IV) may be prepared from known intermediates by, for example, two principal routes. Firstly, they may be prepared, as racemic mixtures, by oxidation with, for example, aqueous hydrogen peroxide and sodium hydrogen carbonate in methanolic solution, of an aldehyde of the formula

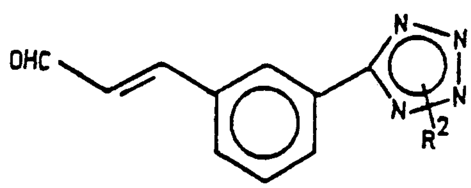

in which $R^2$ is a protecting group and, in its turn, the aldehyde of formula (IV) may be converted to one of formula (V) by reaction with formylmethylenetriphenylphosphorane.

Alternatively, the compounds of formula (IV) may be prepared by oxidation of an epoxy alcohol of the formula

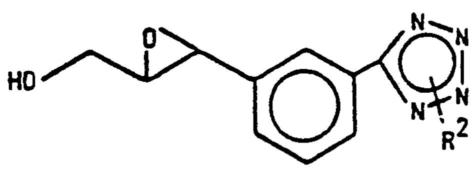

(VI)

with an oxidising agent such as, for example, chromium trioxide in pyridine. Compounds of formula (VI) can be prepared in stereospecific form, the steric configuration being retained on oxidation to provide the aldehyde of formulae (IV) and, ultimately, of formula (V).

Compounds of formula (VI) are prepared from the allyl alcohol

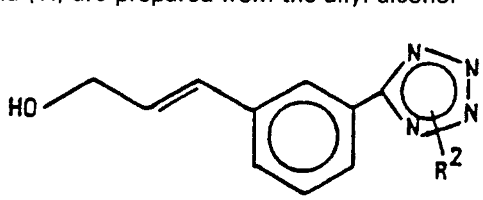

(VII)

using as epoxidising agent a reagent such as titanium isopropoxide-t-butyl hydroperoxide in the presence of L or D diethyl tartrate which yields the S,S or R,R epoxide with the above E olefin. When the Z olefin is used as starting material, the appropriate S,R and R,S stereoisomers result. Compounds of formula (VII)

can be prepared from the appropriate benzaldehyde via a sequence of reactions involving reaction with malonic acid to provide the cinnamic acid derivative, treatment with oxalyl chloride to give the acid chloride, and reduction with a reagent such as lithium tri-t-butoxyaluminohydride. The Z olefin is produced by catalytic reduction using a Lindlar catalyst of the appropriate acetylenic derivative.

Compounds of formula (III) can be prepared from the appropriate compound of formula Br—X—CN by reaction with benzyl mercaptan to give a benzylthio nitrile which, in its turn, can be converted with azide and ammonium chloride to the corresponding tetrazolyl derivative from which the compound of formula (III) is liberated by treatment with sodium in liquid ammonia. A preferred method of making the compound in which —X— is $—(CH_2)_2—$ is from acrylonitrile as starting point using the Michael reaction.

The following scheme shows how compounds of the invention may be prepared ($R^5$ stands for the group

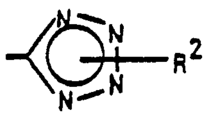

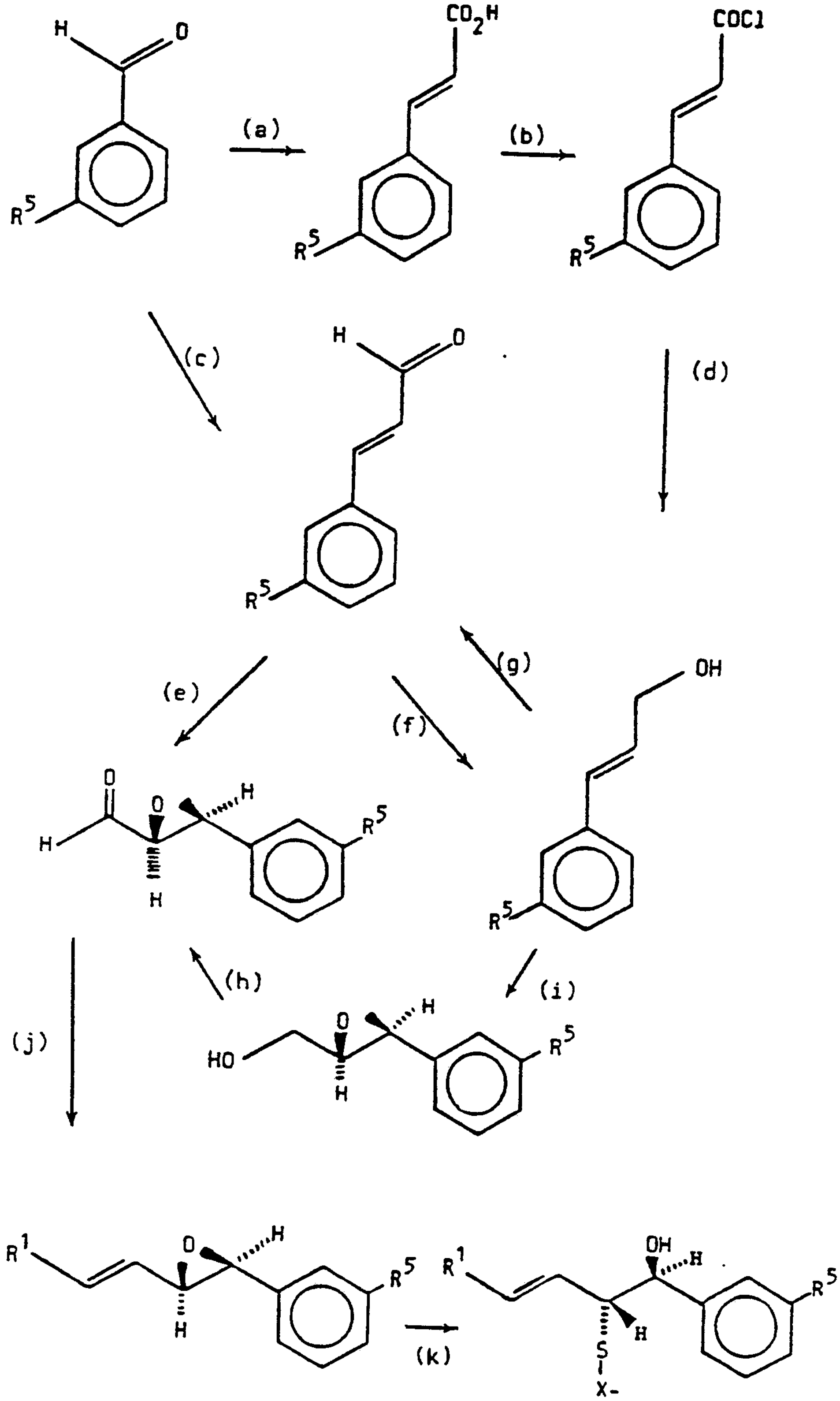


*Key:*

(a) reaction with malonic acid in pyridine and piperidine.
(b) reaction with oxalyl chloride in ether
(c) reaction with formylmethylenetriphenylphosphorane in toluene
(d) reaction with lithium tri-t-butoxyaluminohydride in tetrahydrofuran
(e) reaction with aqueous hydrogen peroxide and sodium hydrogen carbonate in methanolic solution

(f) reaction with sodium borohydride in methanol
(g) reaction with manganese dioxide in dichloromethane
(h) reaction with chromium oxide in pyridine
(i) reaction with titanium isopropoxide t-butyl peroxide in dichloromethane and L-diethyl tartrate
(j) reaction with $R^1CH_2P^+Ph_3Br^-$ in the presence of butyl lithium and in tetrahydrofuran as solvent
(k) reaction with thiol of formula (III).

The compounds of the present invention are pharmacologically active, being leukotriene antagonists as shown by the *in vitro* test on guinea pig ileum segments at a concentrations of from 1 ng/ml to 50 µg/ml, according to the method of Schild (1947) Brit. J. Pharm. *2,* 197—206 (the unprotected compounds of formula (I) described in the following Examples exhibited an $IC_{50}$ against $LTD_4$ of less than $10^{-5}$ molar). Also compounds of the invention are active in the *in vitro* Guinea Pig Pulmonary Function Test of Austen and Drazen (1974) J. Clin. Invest. *53* 1679—1685 at intravenous dosage levels of from 0.05 µg/kg to 5.0 mg/kg and in a modified "Herxheimer" test (Journal of Physiology (London) *117* 251 (1952)) at doses of from 25 to 200 mg/kg. The "Herxheimer" test is based on an $LTD^4$-induced bronchospasm in guinea pigs which closely resembles an asthmatic attack in man. The compounds also show low bronchial irritancy.

The compounds are accordingly indicated for therapeutic use in the treatment of diseases in which leukotrienes are implicated. These include allergic reactions of the pulmonary system in which leukotrienes are thought to be causal mediators of bronchospasm, for example, in allergic lung disorders such as extrinsic asthma and industrial asthmas such as Farmers lung and Pigeon Fanciers lung, and in other inflammatory disorders, for example, associated with acute or chronic infectious diseases such as allergic skin diseases, ectopic and atopic eczemas, psoriasis, contact hypersensitivity and angioneurotic oedema, bronchitis and cystic fibrosis and rheumatic fever.

Thus the invention also includes a pharmaceutical composition comprising a pharmaceutically acceptable diluent or carrier in association with a compound of formula (I) in unprotected form; or a pharmaceutically acceptable salt thereof.

The compounds may be administered by various routes, for examples by the oral or rectal route, topically or parenterally, for example by injection, and especially by inhalation, being usually employed in the form of a pharmaceutical composition. Such compositions are prepared in a manner well known in the pharmaceutical art and normally comprise at least one active compound. In making the compositions of the present invention, the active ingredient will usually be mixed with a carrier, or diluted by a carrier, and/or enclosed within a carrier which may, for example, be in the form of a capsule, sachet, paper or other container. When the carrier serves as a diluent, it may be a solid, semi-solid, or liquid material which acts as a vehicle, excipient or medium for the active ingredient. Thus, the composition may be in the form of tablets, lozenges, sachets, cachets, elixirs, suspensions, aerosols (as a solid or in a liquid medium), ointments containing for example up to 10% by weight of the active compound, soft and hard gelatin capsules, suppositories, injection solutions and suspensions and sterile packaged powders. For administration by inhalation, particular forms of presentation include aerosols, atomisers and vaporisers.

Some examples of suitable carriers are lactose, dextrose, sucrose, sorbitol, mannitol, starches, gum acacia, calcium phosphate, alginates, tragacanth, gelatin, syrup, methyl cellulose, methyl- and propylhydroxybenzoate, talc, magnesium stearate and mineral oil. The compositions of the invention may, as is well known in the art, be formulated so as to provide quick, sustained or delayed release of the active ingredients after administration to the patient.

Where the compositions are formulated in unit dosage form, it is preferred that each unit dosage form contains from 5 mg to 500 mg, for example, from 25 mg to 200 mg. The term "unit dosage form" refers to physically discrete units suitable as unit dosages for human subjects and animals, each unit containing a predetermined quantity of active material calculated to produce the desired therapeutic effect, in association with the required pharmaceutical carrier.

The active compounds are effective over a wide dosage range and, for example, dosages per day will normally fall within the range of from 0.5 to 300 mg/kg, more usually in the range of from 5 to 100 mg/kg. However, it will be understood that the amount administered will be determined by the physician in the light of the relevant circumstances including the condition to be treated, the choice of compound to be administered and the chosen route of administration, and therefore the above dosage ranges are not intended to limit the scope of the invention in any way.

The invention is illustrated by the following Examples. The structure of the compounds prepared was confirmed by I.R. and/or n.m.r. and/or mass spectra.

## Example 1

(A) (i) *3-(Benzylthio)-propionitrile*

Acrylonitrile (21.4 g, 0.4 M) was added dropwise to a solution of benzyl mercaptan (49.6 g, 0.4 M) and triethylamine (4 mls) in methanol (400 mls). This addition led to an exothermic reaction and the temperature of the reaction mixture was allowed to rise from room temperature to 35°C. After 10 minutes the reaction was almost complete as shown by TLC, there being a trace of unreacted thiol still present. Additional acrylonitrile (0.5 g) was then added to consume the last of the thiol. After a further 10 minutes the solution was evaporated to give the title compound 69.0 g as a pale yellow oil.

(ii) *5-[2-(Benzylthio)-ethyl]-1H-tetrazole*

A mixture of 3-(benzylthio)-propionitrile (33.30 g), sodium azide (61.14 g) and ammonium chloride (50.33 g) in dry dimethylformamide (220 mls) was stirred at 120° for 20 hours. Additional sodium azide (24.50 g) and ammonium chloride (20.13 g) was then added, and heating at 120° continued for further 6 hours.

The cooled reaction mixture was poured into a mixture of ice/water and 2 molar hydrochloric acid and extracted with dichloromethane. The organic extracts were in turn extracted with ten per cent sodium carbonate solution, and the basic extracts cautiously acidified with concentrated hydrochloric acid. The resulting precipitate was collected by filtration, washed with water, and dried in vacuo to give the required product as a white solid (28.95 g), m.p. 80—82°C.

(iii) *5-(2-Mercaptoethyl)-1H-tetrazole*

5-[2-(Benzylthio-ethyl]-1H-tetrazole (15.00 g) was added portionwise as a solid to a solution of sodium metal (7.84 g) in liquid ammonia (2—300 mls). The reaction mixture was stirred at 20° under a drying tube (silica gel) for 2 hours and then very cautiously quenched with methanol (100 mls). The excess ammonia was removed under a nitrogen stream and the residue acidified with hydrochloric acid and extracted with ethyl acetate. The organic extracts were dried over magnesium sulphate, filtered and evaporated under reduced pressure to yield the title compound as a white solid (7.15 g), m.p. 104—106°C.

(B)(i) *(3-Cyanocinnamoyl chloride)*

3-Cyanocinnamic acid (200 g) was treated with diethyl ether (3333 ml), oxalyl chloride (100.8 ml) and dimethyl formamide (2 ml) and stirred at room temperature for 5.5 hours in a vessel closed by a silica tube. The solution was decanted from insoluble material and evaporated to dryness to leave a pale yellow to white solid, which was dried at 40—50°/high vacuum to give the acid chloride, melting point 105°C.

(ii) *(3-Hydroxy-1-propenyl)benzonitrile*

$NaBH_4$—$Al_2O_3$ (1219 g) (prepared according to Santaniello, Ponti and Manzocchi, "Synthesis", 1978, 891, but using Woelm Alumina N instead of the type specified: the reagent formed contained 2.04 M.mole of $NaBH_4$ per gram) was stirred in diethyl ether (6340 ml) at room temperature and the acid chloride (200 g) from (B)(i) in ether (7800 ml) was added dropwise during one hour. The temperature rose from 20—27°, and had fallen to 26° one hour after completion of the addition. The mixture was stirred for a total of 1.5 hours after completion of the acid chloride addition. The alumina was filtered off and washed with ether (3 × 2 l), the filtrate was evaporated to dryness to leave an oil which solidified on storage in a refrigerator. The solid alcohol had a melting point <50°C.

(iii) *3-(5-Tetrazolyl)cinnamyl alcohol*

3-Cyanocinnamyl alcohol (292.97 g), dimethylformamide (914 ml), triethylamine hydrochloride (558.7 g) and sodium azide (358.85 g) were added to a flask. The reaction was carried out under nitrogen and after addition of all the reactants heat was applied. The temperature of the reaction mixture reached 100°C 1—2 hours after commencement of heating and the solution was stirred and heated at 100° for 8 hours. The solution was transferred to a 10 litre flask, treated with water (2944 ml), stirred and cooled to 10°C (ice-bath), treated with concentrated HCl (736 ml) and stirred for 2.5 hours while the temperature dropped from 20° to 5°C. The solid was filtered washed with cold water (3 × 2 L and 1 × 1 L) and dried, initially for 1.5 hours in a fluid-bed drier, then at 60°/high vacuum to give 3-(5-tetrazolyl)cinnamyl alcohol, melting point 174°C.

(iv) *(E) 3-[3-(2-Triphenylmethyl-2H-tetrazol-5-yl)phenyl]-2-propenol*

To a solution of 3-[3-(1H-tetrazol-5-yl)phenyl]-2-propenol of (iii) above (2.02 g) in dry dichloromethane (50 ml) was added triethylamine (1.5 ml) followed by triphenylchloromethane (2.8 g) in dry dichloromethane. The solution was stirred at room temperature for 90 minutes, washed with water (50 ml), followed by sodium bicarbonate solution (5%; 50 ml), dried over magnesium sulphate, filtered and evaporated under reduced pressure to give a pale brown viscous oil which crystallized on standing to a cream solid.

(v) *(2S,3S)-3-[3-(2-Triphenylmethyl-2H-tetrazol-5-yl)phenyl]-2,3-epoxypropanol*

L-(+)-Dimethyl tartrate (1.85 g) was added in dry dichloromethane (10 ml) dropwise to a stirred solution of titanium (IV) isopropoxide (3.1 ml) in dry dichloromethane (30 ml) at −20 to −25°C under nitrogen. The solution was stirred for 10 minutes and a solution of 3-[3-(2-triphenylmethyl-2H-tetrazol-5-yl)-phenyl]-2-propenol (4.5 g) in dry dichloromethane (20 ml) was added, followed by a 3.7 M solution of t-butylhydroperoxide in toluene (6.7 ml), both at −20 to −25°C. The pale orange solution was left to stand in a freezer for 3 hours. To the stirred solution was added aqueous tartaric acid (10%; 50 ml) and the mixture stirred for 1 hour, filtered and separated. The dichloromethane layer was dried over magnesium sulphate filtered and evaporated under reduced pressure to give a yellow oil. The oil was dissolved in carbon tetrachloride, washed with water, dried over magnesium sulphate, filtered and evaporated under reduced pressure to give a pale yellow oil. The oil was chromatographed on a silica gel column using diethyl ether

and hexane (2:1) and the required fractions evaporated under reduced pressure to give a colourless crystalline solid.

(vi) *(4S,5S)-5-[3-(2-Triphenylmethyl-2H-tetrazol-5-yl)phenyl]-4,5-epoxy-2-pentenal*

Solid chromium trioxide (5.0 g) was added to a stirred solution of pyridine (7.9 ml) in dry dichloromethane (200 ml) at 5°C. The mixture was stirred for 45 minutes, warming to 13°C, allowing all the chromium trioxide to dissolve, then a solution of the epoxyalcohol of step (ii) (4.61 g) in dry dichloromethane (50 ml) was added rapidly. The dark mixture was stirred for 90 minutes, warming to room temperature, then filtered through a pad of Florisil to remove the chromium salts, and the colourless filtrate was evaporated under reduced pressure to leave a pale yellow oil.

To a solution of the oil (1.8 g) in benzene (75 ml) under nitrogen, was added formylmethylenetriphenylphosphorane (1.34 g) in one portion. The suspension was stirred at room temperature under nitrogen for eight hours, unreacted ylid was filtered off and the filtrate evaporated under reduced pressure to a brown oil. The brown oil was extracted with hot ether, cooled, filtered and evaporated under reduced pressure to give a yellow oil, which crystallised on standing to give a yellow solid.

(vii) *(1S,2S)-5-{3-[2-(1,2-Epoxy)pentadeca-3(E),5(Z)-dienyl]phenyl}-2-triphenylmethyl-2H-tetrazole*

n-Butyl lithium (8.91 ml; 1.5 M) in hexane was added dropwise to a stirred solution of n-decyl triphenylphosphonium bromide (6.07 g) (dried at 80°C under reduced pressure for 16 hours) in dry tetrahydrofuran (130 ml) at −70°C under nitrogen. The clear deep orange solution obtained was stirred for a further 10 minutes at −70°C, then a solution of 5-[3-(2-triphenylmethyl-2H-tetrazol-5-yl)phenyl]-4,5-epoxy-2-pentenal (6.4 g) in dry tetrahydrofuran (75 ml) was added dropwise. The pale yellow solution was stirred at −70°C for 1 hour, allowed to warm to room temperature and evaporated under reduced pressure to a brown oil. The oil was extracted with ether:hexane (1:2; 4 × 200 ml) and the pale hazy extract evaporated under reduced pressure to give the title compound as a yellow oil.

(C)(i) *(1S,2R)-5-{3-[2-(2-(1H)-Tetrazol-5-ylethylthio)-1-hydroxypentadeca-3(E),5(Z)-dienylphenyl}-2-triphenylmethyl-2H-tetrazole*

5-(2-Mercaptoethyl)-1H-tetrazole (73 mg, 0.5 mM) was dissolved in dry tertiary butanol (2 ml) under nitrogen at room temperature. Potassium tertiary butoxide (112.2 mg, 1.0 mM) was added and the resultant suspension stirred at room temperature for 10 minutes. This suspension was then added to (1S,2S)-5 3-[2-(1,2-oxido)pentadeca-3(E),5(Z)-dienyl]phenyl-2-triphenylmethyl-2H-tetrazole also under nitrogen and the resultant mixture stirred at room temperature for 6 hours. Water 10 ml was then added and pH of the solution adjusted to 7 with acetic acid. This aqueous solution was then extracted 3 times with dichloromethane and the combined extracts evaporated to dryness. The resultant colourless oil was chromatographed on a silica column eluted with ethyl acetate. The title compound was obtained cheomatographically pure as a colourless oil, yield 55 mg.

(ii) *(1S,2R)-5-{3-[2-(2-1H-Tetrazol-5-ylethylthio)-1-hydroxypentadeca-3(E),5(Z)-dienyl]phenyl}-1H-tetrazole, disodium salt*

(1S,2R)-5-{3-[2-(2-1H-Tetrazole-5-ylethylthio)-1-hydroxypentadeca-3(E),5(Z)-dienyl]phenyl}-2-triphenylmethyl-2H-tetrazole (30 mg) was dissolved in ether (1 ml), water (1 ml) and 98/100 formic acid (0.5 ml). The resultant solution was stirred at room temperature for 6 hours. The pH of the reaction mixture was then adjusted to 9 using sodium carbonate and extracted three times with ether. The pH of aqueous was then adjusted to 3 using hydrochloric acid and extracted three times with ether. The combined ether extracts were dried and evaporated to yield the free acid of the title compound, yield 12 mg. This was converted to a solution of the disodium salt by reaction with aqueous sodium bicarbonate.

Example 2

This Example illustrates a further preparation of the compound of Example 1C(ii) above, and the isolation of its 3(E), 5(E) isomer.

(i) *(1S,2R)-5-{3-[2-(2-(1H)-Tetrazol-5-ylethylthio)-1-hydroxypentadeca-3(E),5(Z)-dienyl}-2-triphenylmethyl-tetrazole.*

5-(2-Mercaptoethyl)-1H-tetrazole (1.46 g) was dissolved in dry tertiary butanol (15 ml) under nitrogen at room temperature. Potassium tertiary butoxide (2.24 g) was added and the resultant suspension stirred at room temperature for 10 minutes. This suspension was then added to (1S,2S)-5-{3-[2-1,2-oxido) pentadeca-3(E),5(Z)-dienyl]phenyl}-2-triphenylmethyl-tetrazole (1.216 g) also under nitrogen and the resultant mixture stirred at room temperature for 20 hours. The solvent was then removed under reduced pressure and water (120 ml) added, the resultant solution extracted twice with hexane/diethyl ether 50/50 to remove non-acidic non polar impurities. The pH of the aqueous solution was then adjusted to 3 using 2M hydrochloric acid and the suspension extracted five times using diethyl ether. The combined diethyl ether extracts were concentrated to a total volume of 15 ml and this solution of the title compound used in the next experiment.

(ii) *(1S,2R)-5-{3-[2-(2-1H-Tetrazol-5-ylethylthio)-1-hydroxypentadeca-3(E),5(Z)-dienyl]phenyl}-1H-tetrazole.*

The solution of (1S,2R)-5-{3-[2-(2-1H-tetrazole-5-ylethylthio)-1-hydroxypentadeca-3(E),5(Z)-dienyl]phenyl}-2-triphenylmethyl-tetrazole (15 ml) from the previous experiment was stirred under nitrogen. Formic acid 98% (5 ml) and water (5 ml) were added and the solution stirred for a further 6 hours at room temperature. The diethyl ether was then removed under reduced pressure and solid sodium carbonate added to the aqueous solution until the pH was 10. Additional water (15 ml) was then added and the triphenyl carbonate removed by filtration. The aqueous solution was then extracted once with diethyl ether and the pH then adjusted to 3 using 2M hydrochloric acid and extracted five times using chloroform. The combined chloroform extracts were evaporated to dryness to yield the crude product (1.5 g) as an oil.

The oil was then purified by reverse phase high performance liquid chromatography using a 2.5 × 50 cm C18 column eluted with methanol/water:80/20 0.1% acetic acid, the material being processed as ten equal aliquots. The major product was the title compound which after evaporation of the solvent and recrystallization from diethyl ether was obtained as white crystals, mp 100—103°C.

The E,E isomer of title compound was obtained as an impurity which eluted as a separate peak of marginally greater retention time than the title compound.

On evaporation of the solvent (1S,2R)-5-{3-[2-(2-(1H)-tetrazol-5-ylethylthio)-1-hydroxypentadeca-3(E),5(E)-dienyl]phenyl}-1H-tetrazole was obtained as a pale yellow oil.

## Example 3

(A)(i) *2-(Benzylthio)-acetonitrile*

To a stirred solution of benzylthiol (27.28 g) in ethanol (50 ml) under nitrogen was added, dropwise with cooling, triethylamine (22.2 g), then sodium iodide (33 g). After stirring this mixture at room temperature for fifteen minutes, a solution of chloroacetonitrile (16.6 g) in ethanol (50 ml) was added dropwise, with ice-bath cooling. The reaction mixture was stirred at room temperature, under nitrogen, overnight.

The reaction mixture was filtered through Hi-Flow and the filtrates evaporated in vacuo to give a gelatinous residue, which was partitioned between water (200 ml) and diethyl ether (2 × 100 ml). The ether extracts were washed with 2M sodium carbonate (2 × 100 ml) and saturated brine (2 × 100 ml), dried over magnesium sulphate and evaporated in vacuo to give the title compound as a nearly colourless oil (32.54 g).

The following compound was similarly prepared 4-(Benzylthio)-butyronitrile b.p. 172—174°C/mmHg.

(ii) *5-(Benzylthiomethyl)-1H-tetrazole*

A mixture of 2-(benzylthio)-acetonitrile (32.32 g), sodium azide (64.44 g) and ammonium chloride (53.04 g) in dry dimethylformamide (300 mls) was stirred at 100°C for sixteen hours.

The reaction mixture was allowed to cool and then poured into an ice/water mixture (1l) previously acidified to pH2 with 2M hydrochloric acid. The off-white precipitate was collected by filtration, washed with more water on the sinter, and dried in vacuo at 60°C to give the product as an off-white solid (29.5 g), m.p. ~ 100°C.

The following compound was similarly prepared 5-(3-Benzylthiopropyl)-1H-tetrazole, an oil.

(iii) *5-(Mercaptomethyl)-1H-tetrazole*

To a stirred solution of sodium metal (9.9 g) in liquid ammonia (about 400 mls) was added 5-(benzylthiomethyl)-1H-tetrazole (29.5 g). The reaction mixture was stirred at room temperature for two hours and then cautiously quenched with methanol (150 ml) (effervescence). After removing excess ammonia with a fast nitrogen stream the reaction mixture was acidified with concentrated hydrochloric acid and extracted three times with ethyl acetate. The extracts were dried over magnesium sulphate and evaporated in vacuo to give a solid residue which, after washing with hexane, gave the title compound as a white fluffy solid (9.1 g), m.p. 74—76°C.

The following compound was similarly prepared 5-(3-Mercaptopropyl)-1H-tetrazole, an oil.

(B) The intermediates synthesised in (A)(iii) above were used to prepare the following end products by the methods described in Example 1: (1S,2R)-5-{-[2-(2-1H-Tetrazol-5-ylmethylthio)-1-hydroxypentadeca-3(E),5(Z)-dienyl]phenyl}-1H-tetrazole, a yellow oil.

(1S,2R)-5-{-(2-(2-1H-Tetrazol-5-ylpropylthio)-1-hydroxypentadeca-3(E),5(Z)-dienyl]phenyl}-1H-tetrazole, a yellow oil.

The active compounds of the invention are preferably employed in salt form. The following formulations are given by way of example:

## Example 4

*Soft gelatin capsule*

Each soft gelatin capsule contains:

| | |
|---|---|
| Active ingredient | 150 mg |
| Arachis oil | 150 mg |

After mixing together, the blend is filled into soft gelatin capsules using the appropriate equipment.

Example 5

*Hard gelatin capsule*
Each capsule contains

| | |
|---|---|
| Active ingredient | 50 mg |
| PEG 4000 | 250 mg |

The PEG 4000 is melted and mixed with the active ingredient. Whilst still molten the mixture is filled into capsule shells and allowed to cool.

Example 6

*Aerosol*

| | |
|---|---|
| Active ingredient | 10 mg |
| Ethanol | 50 mg |
| Dichlorodifluoromethane (Propellant 12) | 658 mg |
| Dichlorotetrafluoroethane (Propellant 114) | 282 mg |

The active ingredient is dissolved in the ethanol. The concentrate is filled into extruded aluminium cans for inhalation aerosols. The cans are degassed with propellant 12 and sealed with an appropriate metered dose valve. The volume of product expelled per actuation is 50 or 100 µl equivalent to 0.5—1 mg active ingredient.

**Claims for the Contracting States: BE CH DE FR GB IT LI NL SE**

1. A compound of the formula

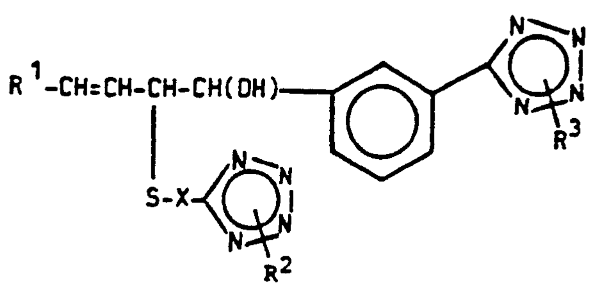

(I)

in which $R^1$ is $C_{7-20}$alkyl or $C_{7-20}$ alkenyl containing 1 to 3 double bonds, $R^2$ and $R^3$ are each hydrogen or a protecting group, and X is $C_{1-6}$ alkylene; or a salt thereof.

2. A compound according to claim 1 in which $R^2$ and $R^3$ are both hydrogen.

3. A compound according to claim 2 in which $R^1$ is of the formula $R^4CH{=}CH—$ where $R^4$ is $C_{6-11}$ alkyl or $CH_3(CH_2)_4CH{=}CHCH_2CH{=}CH_2—$.

4. A compound according to claim 3 in which $R^1CH{=}CH$ in formula (I) is

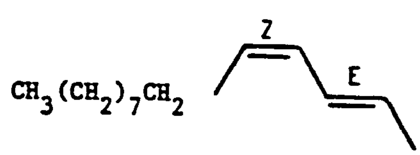

or

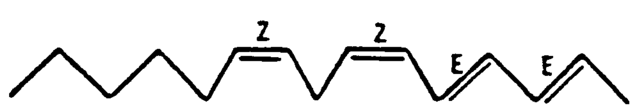

5. A compound according to claim 1 of the formula

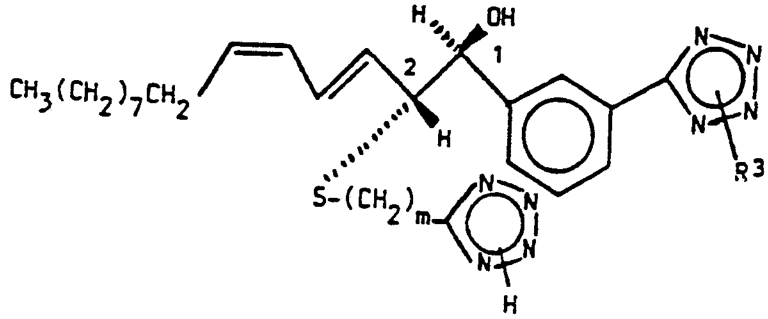

in which $R^3$ is hydrogen or a protecting group and m is 1, 2 or 3; and salts thereof.

6. A compound according to claim 1, which is (1S,2R)-5-3-[2-(2-1H-tetrazol-5-ylethylthio)-1-hydroxy-pentadeca-3(E),5(Z)-dienyl]phenyl-1H-tetrazole, or a pharmaceutically acceptable salt thereof.

7. A pharmaceutical formulation comprising a compound according to claim 2 or a pharmaceutically-acceptable salt thereof in association with a pharmaceutically-acceptable diluent or carrier therefor.

8. A process for producing a compound as defined in claim 1 which comprises reacting a compound of formula

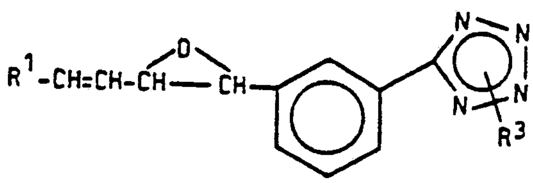

(II)

in which $R^1$ is as defined in claim 1 and $R^3$ is a protecting group, with a thiol of formula

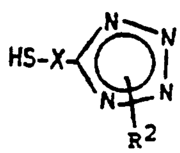

(III)

in which $R^2$ is hydrogen or a protecting group, optionally followed by removal of an $R^2$ or $R^3$ protecting group.

9. A compound according to claim 2 or a pharmaceutically-acceptable salt thereof, for use as a pharmaceutical.

**Claims for the Contracting State: AT**

1. A process for preparing a compound of the formula

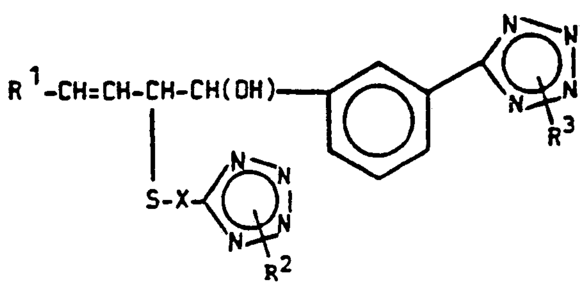

(I)

in which $R^1$ is $C_{7-20}$alkyl or $C_{7-20}$ alkenyl containing 1 to 3 double bonds, $R^2$ and $R^3$ are each hydrogen or a protecting group, and X is $C_{1-6}$ alkylene; or a salt thereof; which comprises reacting a compound of the formula

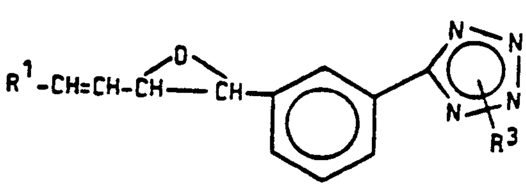

(II)

in which $R^1$ is defined above and $R^3$ is a protecting group, with a thiol of formula

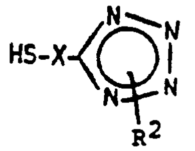

(III)

in which $R^2$ is hydrogen or a protecting group, optionally followed by removal of an $R^2$ or $R^3$ protecting group.

2. A process according to claim 1 for preparing a compound in which $R^2$ and $R^3$ are both hydrogen.

3. A process according to claim 2 for preparing a compound in which $R^1$ is of the formula $R^4CH{=}CH—$ where $R^4$ is $C_{6-11}$ alkyl or $CH_3(CH_2)_4CH{=}CHCH_2CH{=}CH_2—$.

4. A process according to claim 3 for preparing a compound in which $R^1CH{=}CH$ in formula (I) is

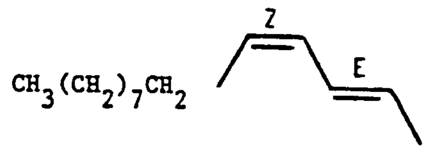

or

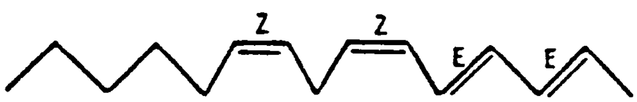

5. A process according to claim 1 for preparing a compound of the formula

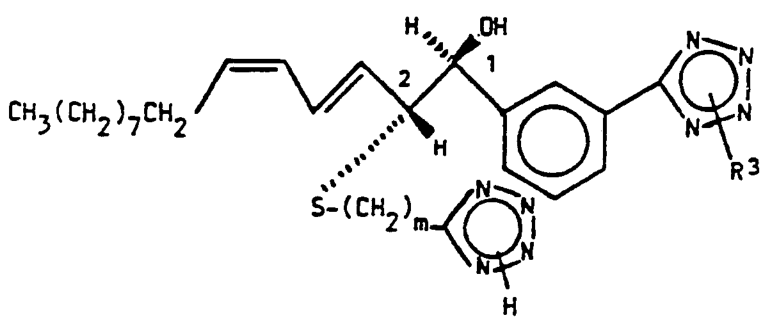

in which $R^3$ is hydrogen or a protecting group and m is 1, 2 or 3; and salts thereof.

6. A process according to claim 1 for preparing (1S,2R)-5-{3-[2-(2-1H-Tetrazol-5-ylethylthio)-1-hydroxy-pentadeca-3(E),5(Z)-dienyl]phenyl}-1H-tetrazole, or a pharmaceutically acceptable salt thereof.

**Patentansprüche für die Vertragsstaaten: BE CH DE FR GB IT LI NL SE**

1. Verbindung der Formel

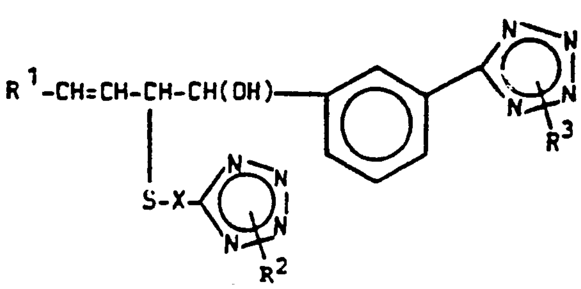

(I)

worin $R^1$ für $C_{7-20}$-Alkyl oder $C_{7-20}$-Alkenyl, das 1 bis 3 Doppelbindungen enthält, und $R^2$ und $R^3$ jeweils für Wasserstoff oder eine Schutzgruppe und X für $C_{1-6}$-Alkylen stehen, oder ein Salz davon.

2. Verbindung nach Anspruch 1, worin $R^2$ und $R^3$ beide Wasserstoff bedeuten.

3. Verbindung nach Anspruch 2, worin $R^1$ die Formel hat $R^4CH{=}CH{-}$, worin $R^4$ für $C_{6-11}$-Alkyl oder $CH_3(CH_2)_4CH{=}CHCH_2CH{=}CH_2{-}$ steht.

4. Verbindung nach Anspruch 3, worin $R^1{-}CH{=}CH$ in der Formel (I) bedeutet

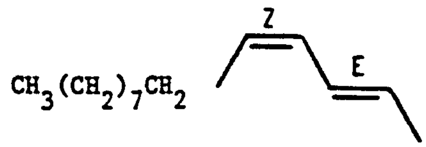

oder

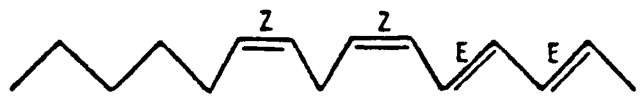

5. Verbindung nach Anspruch 1 der Formel

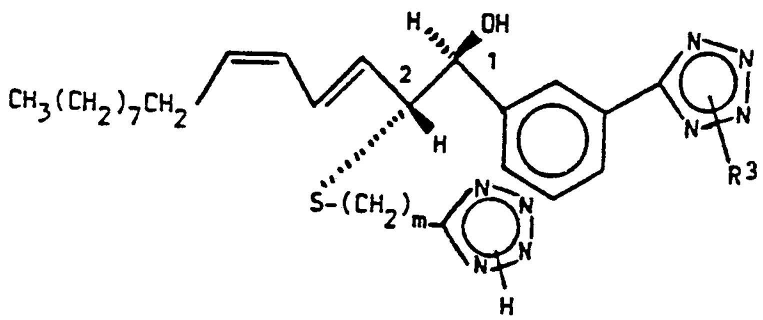

worin $R^3$ für Wasserstoff oder eine Schutzgruppe und m für die Zahl 1, 2 oder 3 stehen, und ihre Salze.

6. Verbindung nach Anspruch 1, bei der es sich handelt um (1S,2R)-5-{3-[2-(2-1H-Tetrazol-5-ylethylthio)-1-hydroxypentadeca-3(E),5(Z)-dienyl]phenyl}-1H-tetrazole oder ein pharmazeutisch akzeptables Salz davon.

7. Pharmazeutische Formulierung, die eine Verbindung nach Anspruch 2 oder ein pharmazeutisch akzeptables Salz derselben in Assoziation mit einem pharmazeutisch akzeptablen Verdünnungsmittel oder Träger dafür enthält.

8. Verfahren zur Herstellung einer Verbindung, wie sie in Anspruch 1 definiert ist, das umfaßt die Umsetzung einer Verbindung der Formel

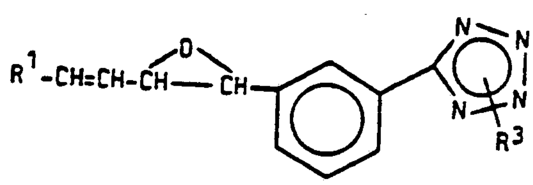

(II)

worin $R^1$ wie in Anspruch 1 definiert ist und $R^3$ eine Schutzgruppe darstellt, mit einem Thiol der Formel

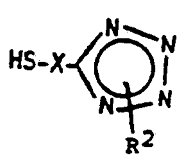

(III)

worin $R^2$ Wasserstoff oder eine Schutzgruppe darstellt, woran sich gegebenenfalls die Entfernung einer $R^2$- oder $R^3$-Schutzgruppe anschließt.

9. Verbindung nach Anspruch 2 oder eines pharmazeutisch akzeptablen Salzes derselben für die Verwendung als Arzneimittel.

**Patentansprüche für den Vertragsstaat: AT**

1. Verfahren zur Herstellung einer Verbindung der Formel

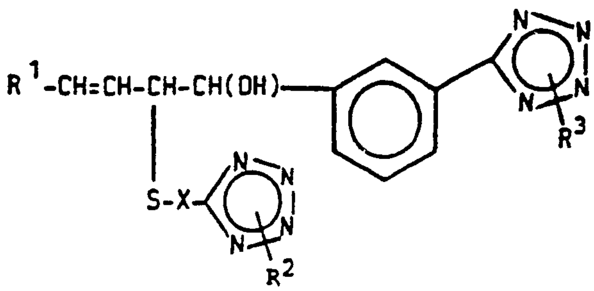

(I)

worin $R^1$ für $C_{7-20}$-Alkyl oder $C_{7-20}$-Alkenyl, das 1 bis 3 Doppelbindungen enthält, $R^2$ und $R^3$ jeweils für Wasserstoff oder eine Schutzgruppe und X für $C_{1-6}$-Alkylen stehen, oder eines Salzes derselben, das umfaßt die Umsetzung einer Verbindung der Formel

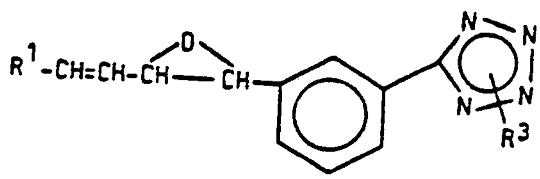

(II)

worin $R^1$ wie oben definiert ist und $R^3$ eine Schutzgruppe darstellt, mit einem Thiol der Formel

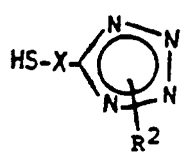

(III)

worin $R^2$ für Wasserstoff oder eine Schutzgruppe steht, woran sich gegebenenfalls die Entfernung einer $R^2$- oder $R^3$-Schutzgruppe anschließt.

2. Verfahren nach Anspruch 1 zur Herstellung einer Verbindung, in der $R^2$ und $R^3$ beide Wasserstoff bedeuten.

3. Verfahren nach Anspruch 2 zur Herstellung einer Verbindung, in der $R^1$ die Formel hat $R^4$—CH=CH—, worin $R^4$ für $C_{6-11}$-Alkyl oder $CH_3(CH_2)_4CH=CHCH_2CH=CH_2$— steht.

4. Verfahren nach Anspruch 3 zur Herstellung einer Verbindung, in der $R^1$ —CH=CH in der Formel (I) steht für

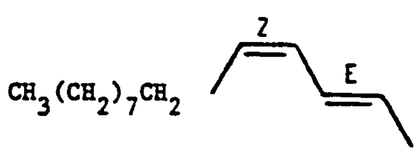

oder

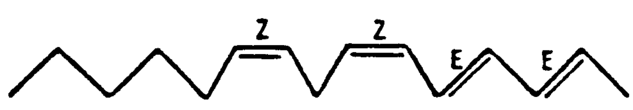

5. Verfahren nach Anspruch 1 zur Herstellung einer Verbindung der Formel

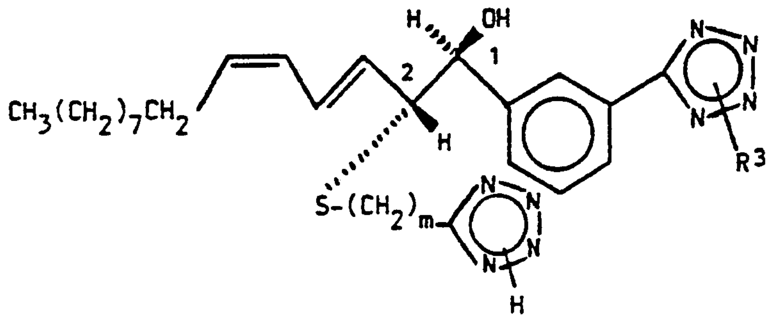

worin $R^3$ für Wasserstoff oder eine Schutzgruppe und m für die Zahl 1, 2 oder 3 stehen, sowie ihrer Salze.

6. Verfahren nach Anspruch 1 zur Herstellung von (1S,2R)-5-{3-[2-(2-1H-Tetrazol-5-ylethylthio)-1-hydroxypentadeca-3(E),5(Z)-dienyl]phenyl}-1H-tetrazol oder einem pharmazeutisch akzeptablen Salz derselben.

**Revendications pour les Etats contractants: BE CH DE FR GB IT LI NL SE**

1. Composé de formule

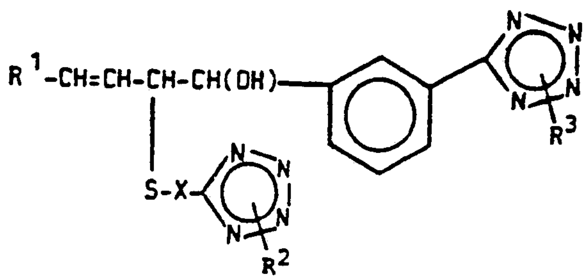

(I)

dans laquelle $R^1$ représente un groupe alkyle en $C_7$—$C_{20}$ ou un groupe alcényle en $C_7$—$C_{20}$ contenant 1 à 3 liaisons doubles, $R^2$ et $R^3$ représentent chacun un atome d'hydrogène ou un groupe protecteur et X représente un groupe alkylène en $C_1$—$C_6$; ou un de ses sels.

2. Composé selon la revendication 1, dans lequel $R^2$ et $R^3$ représentent tous deux un atome d'hydrogène.

3. Composé selon la revendication 2, dans lequel $R^1$ répond à la formule $R^4CH{=}CH$—, où $R^4$ représente un groupe alkyle en $C_6$—$C_{11}$ ou $CH_3(CH_2)_4CH{=}CHCH_2CH{=}CH_2$—.

4. Composé selon la revendication 3, dans lequel $R^1CH{=}CH$ dans la formule (I) représente

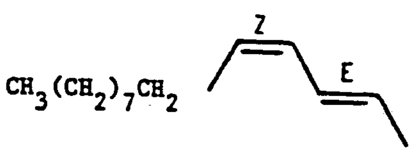

et

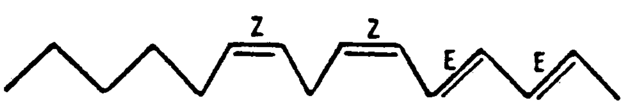

5. Composé selon la revendication 1, de formule

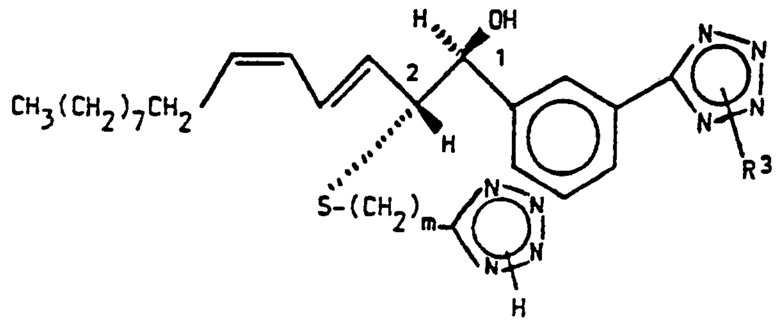

dans laquelle $R^3$ représente un atome d'hydrogène ou un groupe protecteur et m représente 1, 2 ou 3; ainsi que ses sels.

6. Composé selon la revendication 1, à savoir le (1S,2R)-5-{3-[2-(2-1H-tétrazol-5-yléthylthio)-1-hydroxypentadéca-3(E),5(Z)-diényl]phényl}-1H-tétrazole, ou un de ses sels pharmaceutiquement acceptables.

7. Formulation pharmaceutique comprenant un composé selon la revendication 2 ou un de ses sels pharmaceutiquement acceptables, en association avec un diluant ou un support pharmaceutiquement acceptable pour ce composé.

8. Procédé de préparation d'un composé tel que défini dans la revendication 1, ce procédé consistant à faire réagir un composé de formule

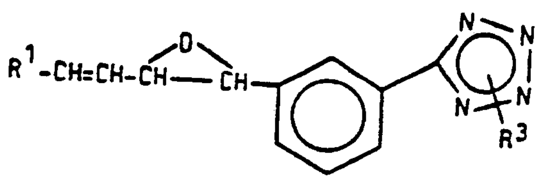

(II)

dans laquelle $R^1$ a la signification définie dans la revendication 1 et $R^3$ est un groupe protecteur, avec un thiol de formule

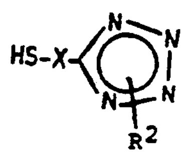

(III)

dans laquelle $R^2$ représente un atome d'hydrogène ou un groupe protecteur, cette réaction étant éventuellement suivie de l'élimination d'un groupe protecteur $R^2$ ou $R^3$.

9. Composé selon la revendication 2 ou un de ses sels pharmaceutiquement acceptables, pour être utilisé comme produit pharmaceutique.

**Revendications pour l'Etat contractant: AT**

1. Procédé de préparation d'un composé de formule

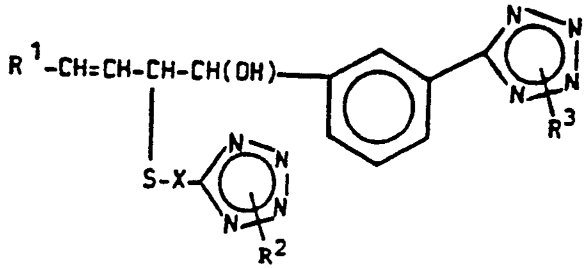

(I)

dans laquelle $R^1$ représente un groupe alkyle en $C_7$—$C_{20}$ ou un groupe alcényle en $C_7$—$C_{20}$ contenant 1 à 3 liaisons doubles, $R^2$ et $R^3$ représentent chacun un atome d'hydrogène ou un groupe protecteur et X

représente un groupe alkylène en $C_1$—$C_6$; ou un de ses sels; ce procédé consistant à faire réagir un composé de formule

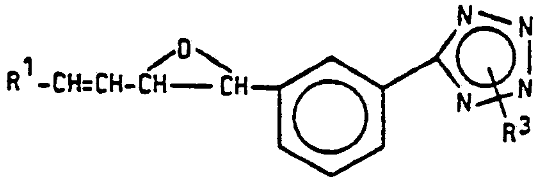

(II)

dans laquelle $R^1$ a la signification définie ci-dessus et $R^3$ est un groupe protecteur, avec un thiol de formule

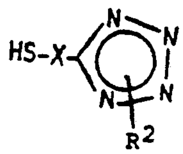

(III)

dans laquelle $R^2$ représente un atome d'hydrogène ou un groupe protecteur, cette réaction étant éventuellement suivie de l'élimination d'un groupe protecteur $R^2$ ou $R^3$.

2. Procédé selon la revendication 1, pour préparer un composé dans lequel $R^2$ et $R^3$ représentent tous deux un atome d'hydrogène.

3. Procédé selon la revendication 2, pour préparer un composé dans lequel $R^1$ répond à la formule $R^4CH=CH$—, où $R^4$ représente un groupe alkyle en $C_6$—$C_{11}$ ou $CH_3(CH_2)_4CH=CHCH_2CH=CH_2$—.

4. Procédé selon la revendication 3, pour préparer un composé dans lequel $R^1CH=CH$ dans la formule (I) représente

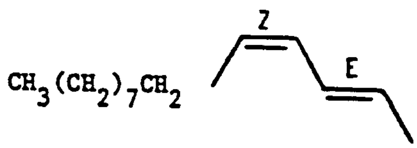

et

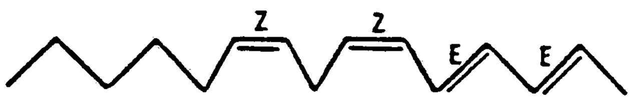

5. Procédé selon la revendication 1, pour préparer un composé de formule

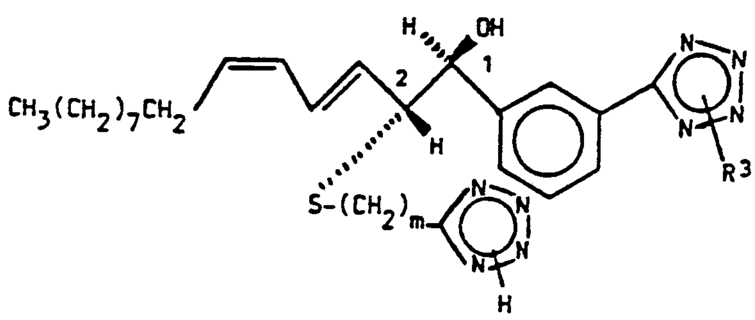

dans laquelle $R^3$ représente un atome d'hydrogène ou un groupe protecteur et m représente 1, 2 ou 3; ainsi que ses sels.

6. Procédé selon la revendication 1, pour préparer le (1S,2R)-5-{3-[2-(2-1H-tétrazol-5-yléthylthio)-1-hydroxypentadéca-3(E),5(Z)-diényl]phényl}-1H-tétrazole, ou un de ses sels pharmaceutiquement acceptables.